Europäisches Patentamt

**European Patent Office**

Office européen des brevets

(11) Veröffentlichungsnummer: **0 013 373**
**B1**

(12) **EUROPÄISCHE PATENTSCHRIFT**

(45) Veröffentlichungstag der Patentschrift:
**18.11.81**

(21) Anmeldenummer: **79105131.1**

(22) Anmeldetag: **13.12.79**

(51) Int. Cl.³: **C 07 D 307/08**

(54) Verfahren zur Herstellung von Tetrahydrofuran.

(30) Priorität: **28.12.78 DE 2856455**

(43) Veröffentlichungstag der Anmeldung:
**23.07.80 Patentblatt 80/15**

(45) Bekanntmachung des Hinweises auf die Patenterteilung:
**18.11.81 Patentblatt 81/46**

(84) Benannte Vertragsstaaten:
**BE DE FR GB IT NL**

(56) Entgegenhaltungen:
**DE-A-2 456 780**

**CHEMICAL ABSTRACTS, Band 84, Nr. 9, 1. März 1976, Zusammenfassung Columbus, Ohio, US,**

(73) Patentinhaber: **BASF Aktiengesellschaft, Carl-Bosch-Strasse 38, D-6700 Ludwigshafen (DE)**

(72) Erfinder: **Hartig, Jürgen, Dr. Dipl.-Chem., In der Schleit 9, D-6718 Gruenstadt (DE)**
Erfinder: **Weitz, Hans-Martin, Dr. Dipl.-Chem., Auf dem Koeppel 40, D-6702 Bad Duerkheim 1 (DE)**

ACTORUM AG.

## Verfahren zur Herstellung von Tetrahydrofuran

Es ist bekannt, dass Tetrahydrofuran (THF) nach einer Reihe unterschiedlicher Verfahren hergestellt werden kann, von denen die wichtigeren die Dehydratisierung von 1,4-Butandiol und die katalytische Hydrierung von Furan sind. Das meiste Tetrahydrofuran wird in der Tat nach einer Mehrstufenfolge hergestellt, ausgehend von der Reaktion des Acetylens und des Formaldehyds unter Bildung von Butindiol, Hydrierung des Butindiols zu Butandiol, welches seinerseits zu Tetrahydrofuran dehydratisiert wird.

Darüber hinaus kann Tetrahydrofuran durch katalytische Hydrierung von Maleinsäure, Fumarsäure und Bernsteinsäure, ihren entsprechenden Anhydriden und Esterderivaten sowie aus Butyrolacton hergestellt werden.

Neuerlich sind Carbonsäureester des 1,4-Butandiols mit Hilfe verschiedener Verfahren im technischen Massstab zugänglich geworden, die ebenfalls, durch sogenannte Dehydroacyloxylierung, d.h. Abspaltung von Carbonsäure unter Verätherung Tetrahydrofuran liefern können.

Hierzu sind schon, z.B. in der GB-PS 1 170 222 DE-OS 2 653 136 Vorschläge gemacht worden.

Diese Vorschläge beziehen sich sowohl auf Flüssigphasen- wie auf Dampf-(Gas-)phasenreaktionen. Als Katalysatoren sind u.a. Aluminiumoxid, Kieselsäure und gemischte derartige Oxide angegeben.

Es scheint, dass man mit Aluminiumoxid keinen besonderen Erfolg hatte, weil in der DE-OS 2 653 136 die Verwendung von Aluminiumoxid mit einem Gehalt an Wolframoxid als Verbesserung empfohlen wird.

Tatsächlich konnte festgestellt werden, dass handelsübliche Aluminiumoxide, die als Katalysatoren gebräuchlich sind bzw. empfohlen werden, für die Herstellung von THF aus Butandioldiacetat in der Gasphase schlecht geeignet oder ungeeignet sind.

Es wurde nun das im voranstehenden Patentanspruch beschriebene Verfahren gefunden, das also auf der Verwendung eines speziell hergestellten Aluminiumoxids beruht.

Hierzu ist daran zu erinnern, dass Aluminiumhydroxide sowohl aus saurer wie aus basischer Lösung fällbar sind, wobei zunächst Gele entstehen, die beim Erhitzen allmählich entwässert werden und schliesslich zu verschiedenen Modifikationen von Aluminiumoxid kristallisieren. Ausserdem spielt bei der Erzielung wirksamer Katalysatoren das Säure-Anion eine erhebliche Rolle, denn die Fällung aus salpetersaurer Lösung führt im Gegensatz zu der erfindungsgemässen Fällung aus schwefelsaurer Lösung nicht zu vorteilhaften Katalysatoren.

Wie die Oberflächenbestimmung solcher Oxide zeigt, handelt es sich meist um fehlstellenreiche, allenfalls mikrokristalline Modifikationen, die zudem von der Herstellung her in bestimmter Weise verunreinigt (dotiert) sind.

Die Katalysatoren werden somit durch Fällung einer wässrigen Aluminiumsulfatlösung mit basisch wirkenden Mitteln, wie die Hydroxide oder Carbonate der Alkali- oder Erdalkalimetalle, Ammoniak oder basische Ammoniumsalze, und Erhitzen der Fällung bei Temperaturen von 70 bis 120°C erhalten. Bevorzugte basisch wirkende Mittel der genannten Art sind schwach basische Mittel, wie die Carbonate, insbesondere das Ammoniumcarbonat. Man trocknet das Aluminiumhydroxid-Gel vorzugsweise bei Temperaturen von 80 bis 110°C unter vermindertem Druck.

Zur Benutzung der Katalysatoren in dem besprochenen Verfahren ist im einzelnen das folgende zu sagen: Ester des Butandiols i.S.d. Erfindung sind vornehmlich die Ester der Ameisensäure, Essigsäure und Propionsäure. Aus wirtschaftlichen Gründen spielen Ester höherer Fettsäuren keine besondere Rolle.

Die Reaktion in der Gasphase beginnt bei Temperaturen um 175°C in merklichem Masse; wegen des Siedepunkts der Ester bei atmosphärischem Druck wird i.a. bei 200 bis 320°C, bevorzugt 280 bis 320°C gearbeitet. Ein geringfügig höherer Druck, z.B. bis 5 bar, kann im Einzelfall zu Einsparungen bei der Dimensionierung der Anlagenteile führen, sofern die Temperatur den gewünschten Aggregatzustand sichert.

Es kann eine Katalysatoranordnung im Festbett oder als Wirbelschicht gewählt werden.

Die Reaktion wird in Gegenwart von wenigstens 1 Mol Wasserdampf je Mol Ester ausgeführt; empfehlenswert ist ein Wasserdampfüberschuss von bis zu 15 Mol.

Die Aufarbeitung der Reaktionsgase geschieht wie üblich; in manchen Fällen kann ein unvollständiger Umsatz mit Rückführung von Ausgangsmaterial gewählt werden, jedoch ist der erfindungsgemäss leicht erzielbare, praktisch vollständige Umsatz vorzuziehen.

Herstellbeispiel A

für einen nicht erfindungsgemässen Katalysator

1 Mol (374,1 g) Aluminiumnitrat (Al(NO$_3$)$_3$ · 9 H$_2$O) werden in 1,5 kg destilliertem Wasser gelöst und in einem 6-l-Rührkolben auf 60°C erhitzt. 300,3 g Ammoniumcarbonat werden in 1,78 kg destilliertem Wasser aufgelöst und innerhalb 50 Minuten zu der vorgelegten Aluminiumnitratlösung unter Rühren allmählich zugefügt. Am Ende der Zugabe hat die Lösung einen pH-Wert von 7,5. Der entstandene Niederschlag wird abzentrifugiert und dreimal mit 0,5 kg Wasser aufgeschlämmt und jeweils zentrifugiert. Man trocknet bei 100°C während 12 Stunden im Wasserstrahlvakuum, zerkleinert und trocknet 18 Stunden nach. Der trockene Rückstand (87 g) wird nachzerkleinert und eine Kornfraktion von 0,1 bis 0,5 mm Maschenweite ausgesiebt.

Herstellbeispiel B

für einen erfindungsgemässen Katalysator

Man verfährt wie im Beispiel 1 beschrieben, wobei man jedoch 1 Mol (666,4 g) Aluminiumsulfat $(Al_2(SO_4)_3 \cdot 18H_2O)$ in 1,2 kg destilliertem Wasser löst und zu dieser Lösung bei 60°C innerhalb von 60 Minuten eine Lösung von 592,0 g Ammoniumcarbonat in 2,5 kg destilliertem Wasser gibt. Man erhält schliesslich 184,1 g Rückstand, aus dem wiederum eine Kornfraktion von 0,1 bis 0,5 mm ausgesiebt wird.

Beispiel 1

20 g des Katalysators aus Beispiel B werden in ein senkrecht eingespanntes, als Modell eines Wirbelschichtreaktors dienendes Quarz-Reaktionsrohr (Länge 160 mm, Durchmesser 62 mm) eingefüllt. Am unteren Ende des Rohres befindet sich eine Quarz-Fritte, am oberen Ende eine absteigende Leitung zu einem nachgeschalteten Kühler sowie Abscheider und Kühlfalle. Es werden stündlich 120 ml (125 g) Butandiol-1,4-diacetat und 34,2 ml Wasser (Molverhältnis Diacetat zu $H_2O = 1:2,65$) verdampft und bei 285 bis 300°C von unten in den Reaktor eingeleitet. Nach 115 Minuten wird der Versuch abgebrochen. Es werden 302,2 g flüssige Reaktionsprodukte erhalten, die 19,23 Gew.-% THF und 32,07 Gew.-% Butandioldiacetat enthalten. Aus dem Abgas werden 4,16 mMol Butadien isoliert. Der Umsatz ist demnach 59,5 Mol-%, d.h. die Ausbeute an THF beträgt, bezogen auf den Umsatz 98,6%.

Vergleichsversuch

Der Reaktor wird mit 20 g Katalysator gemäss Herstellbeispiel A befüllt. Über 2 Stunden werden 261 ml (271,4 g) Butandioldiacetat und 84 g Wasser (Molverhältnis 1:3) bei 292 bis 307°C zugeführt. Man erhält 304,5 g flüssige Reaktionsprodukte, die 69,8 g Diacetat und 10 g THF enthalten. Aus der Kühlfalle wird 28,8 g Butadien isoliert. Bei einem Umsatz von 74,3% beträgt die Umwandlung zu THF 8,8%.

**Patentansprüche**

1. Verfahren zur Herstellung von Tetrahydrofuran durch Erhitzen eines Carbonsäurediesters des 1,4-Butandiols in der Gasphase in Gegenwart eines Aluminiumoxidkatalysators und von mindestens einem Mol Wasserdampf je Mol des Carbonsäurediesters auf eine Temperatur oberhalb von etwa 160°C, dadurch gekennzeichnet, dass man einen Aluminiumoxidkatalysator verwendet, wie er durch Trocknen eines aus wässriger Aluminiumsulfatlösung mit einem basisch wirkenden Mittel gefällten Aluminiumhydroxid-Gels bei Temperaturen von 70 bis 120°C erhalten wird.

2. Verfahren nach Anspruch 1, dadurch gekennzeichnet, dass man einen Aluminiumoxidkatalysator verwendet, bei dessen Herstellung das Aluminiumhydroxid-Gel unter vermindertem Druck auf Temperaturen von 80 bis 110°C erhitzt wurde.

**Claims**

1. A process for the preparation of tetrahydrofuran by heating a carboxylic acid diester of butane-1,4-diol in the gas phase at above about 160°C in the presence of an aluminum oxide catalyst and of at least one mole of steam per mole of the carboxylic acid diester, characterized in that an aluminum oxide catalyst is used which has been obtained by drying, at 70 to 120°C, an aluminum hydroxide gel precipitated from an aqueous aluminum sulfate solution by means of a basic agent.

2. A process as claimed in claim 1, characterized in that an aluminum oxide catalyst is used, in the production of which the aluminum hydroxide gel has been heated at 80 to 110°C under reduced pressure.

**Revendications**

1. Procédé pour la préparation de tétrahydrofuranne par chauffage d'un diester carboxylique du 1,4-butane-diol en phase gazeuse, en présence d'un catalyseur à base d'oxyde d'aluminium et d'au moins 1 mol de vapeur d'eau par mol du diester carboxylique à une température supérieure à 160°C environ, caractérisé en ce qu'on utilise un catalyseur à l'oxyde d'aluminium obtenu par séchage, à une température de 70 à 120°C, d'un gel d'hydroxyde d'aluminium résultant de la précipitation à partir d'une solution aqueuse de sulfate d'aluminium avec un agent à action basique.

2. Procédé selon la revendication 1, caractérisé en ce qu'on utilise un catalyseur à l'oxyde d'aluminium pour la préparation duquel on a chauffé le gel d'hydroxyde d'aluminium sous pression réduite à une température de 80 à 110°C.